# EUROPEAN PATENT APPLICATION

(11) **EP 4 544 986 A1**
(43) Date of publication of application: **30.04.2025**
(21) Application number: 23826905.4
(22) Date of filing: 26.05.2023
(51) Int. Cl.: A61B 3/02, G02C 7/06, G02C 13/00

(54) **SENSITIVITY EVALUATION METHOD AND METHOD FOR MANUFACTURING ONE PAIR OF EYEGLASS LENSES**

(30) Priority: 22.06.2022 JP 2022100238
(71) Applicant: Nikon-Essilor Co., Ltd., Tokyo 130-0026 (JP)
(72) Inventor: CHO, Sungjin, Tokyo 130-0026 (JP)
(74) Representative: Viering, Jentschura & Partner mbB Patent- und Rechtsanwälte
(86) International application number: PCT/JP2023/019716
(87) International publication number: WO 2023/248717

(57) **Abstract**

One of both eyes (e.g., a right eye) is made to view a reference blurred image (Cp), the other of both eyes (e.g., a left eye) is made to view blurred images (Cp+1, Cp+2, ...), and information on sensitivity to a difference in blur amount between the blurred images (Cp+1, Cp+2, ...) that the other eye was made to view and the reference blurred image (Cp) that the one eye was made to view is acquired. Additionally, the other eye is made to view the reference blurred image (Cp), the one eye is made to view the blurred images (Cp+1, Cp+2, ...), and information on sensitivity to a difference in blur amount between the blurred images (Cp+1, Cp+2, ...) that the one eye was made to view and the reference blurred image (Cp) that the other eye was made to view is acquired.

## Description

### TECHNICAL FIELD

The present invention relates to a sensitivity evaluation method and a method for manufacturing a pair of eyeglass lenses.

### BACKGROUND ART

Binocular vision is known to improve visual acuity with an effect called binocular summation (also called binocular advantage) which enhances contrast sensitivity compared to monocular vision. As for progressive refractive power lenses, which are a type of eyeglass lens (e.g., see Patent Document 1), overlaying areas with clear sight (areas with small aberrations) within distance portions having refractive power suitable for distance viewing for both eyes allows for clearer sight of objects through binocular vision. The effect called binocular summation, however, varies among individuals, and eyeglass lenses should be designed in accordance with the wearer's sensitivity.

### PRIOR ART DOCUMENT

### PATENT DOCUMENT

Patent Document 1: WO 2006/001409

### SUMMARY OF THE INVENTION

A sensitivity evaluation method of an aspect of the invention comprises: having a right eye and a left eye each view an image with different blur amounts; and acquiring information on sensitivity to a difference in blur amount between the images that the right and left eyes were each made to view.

A method for manufacturing a pair of eyeglass lenses of an aspect of the invention is for manufacturing a pair of eyeglass lenses consisting of a right-eye eyeglass lens for use on a right eye and a left-eye eyeglass lens for use on a left eye, and the method comprises: having a right eye and a left eye each view an image with different blur amounts; acquiring information on sensitivity to a difference in blur amount between the images that the right and left eyes were each made to view; designing the right-eye and left-eye eyeglass lenses based on the information on sensitivity to the difference in blur amount; and manufacturing the right-eye and left-eye eyeglass lenses based on the design.

### BRIEF DESCRIPTION OF THE DRAWINGS

Figure 1 is a schematic diagram showing a pair of eyeglass lenses of an embodiment;
Figure 2 is a top cross-sectional view of the pair of eyeglass lenses;
Figure 3 is a block diagram showing an eyeglass lens order placing and receiving system;
Figure 4 is a flowchart showing a flow of a method for manufacturing the pair of eyeglass lenses;
Figure 5 shows an example of an order placement screen;
Figure 6 (A) shows an original image before being processed into blurred images;
Figure 6 (B) shows blurred image examples;
Figure 7 is a block diagram showing an evaluation apparatus;
Figure 8 is a flowchart showing a flow of a sensitivity evaluation method;
Figure 9 is a conceptual diagram showing an example in which a sensitivity evaluation is performed;
Figure 10 is a flowchart showing a flow of an eyeglass lens design method;
Figure 11 (A) shows the distribution of astigmatism in an eyeglass lens designed with a first lens design condition;
Figure 11 (B) shows the distribution of astigmatism in the eyeglass lens designed with a second lens design condition;
Figure 11 (C) shows the distribution of astigmatism in the eyeglass lens designed with a third lens design condition;
Figure 12 is a graph showing the magnitude of astigmatism in a distance portion of an eyeglass lens for each lens design condition for comparison;
Figure 13 is a graph showing the magnitude of astigmatism in distance portions of eyeglass lenses designed with the first lens design condition;
Figure 14 is a graph showing the magnitude of astigmatism in the distance portions of the eyeglass lenses designed with the second lens design condition; and
Figure 15 is a conceptual diagram showing a variation in which a sensitivity evaluation is performed.

### MODES OF EMBODYING THE INVENTION

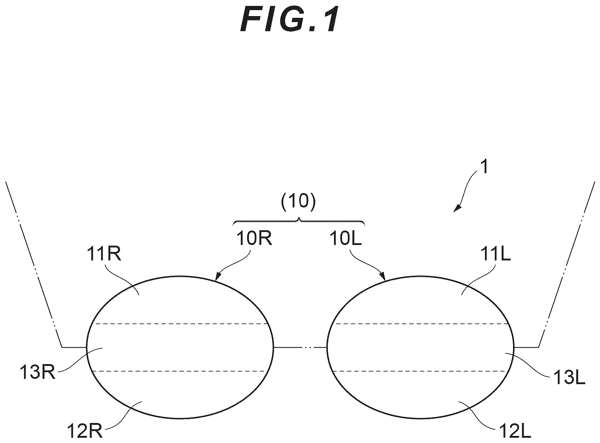

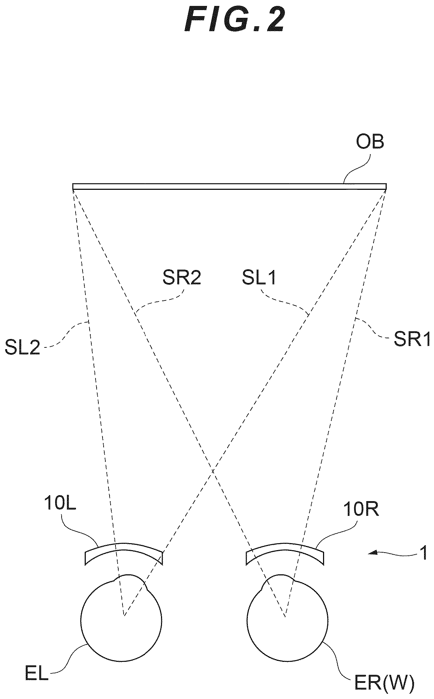

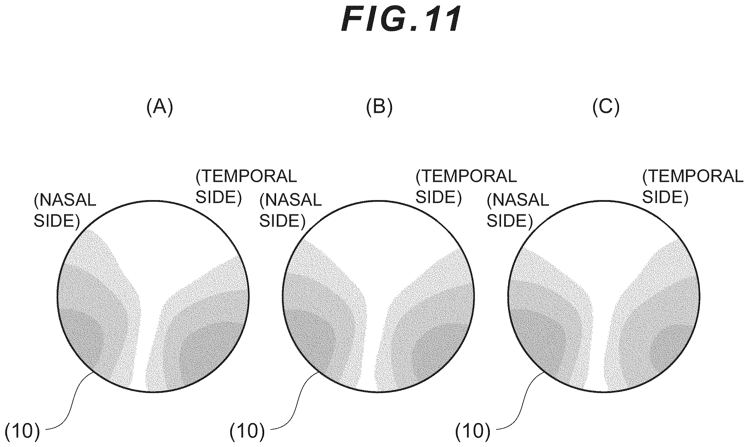

A preferred embodiment of the invention will be described below. Figures 1 and 2 schematically show a pair of eyeglass lenses 1 of the embodiment. As shown in Figures 1 and 2, the pair of eyeglass lenses 1 consists of a right-eye eyeglass lens 10R for use on a right eye ER and a left-eye eyeglass lens 10L for use on a left eye EL. In the embodiment, the right-eye eyeglass lens 10R and the left-eye eyeglass lens 10L are sometimes collectively and simply referred to as the eyeglass lens 10. The eyeglass lens 10 is also referred to as the progressive refractive power lens. The positional relationship of the eyeglass lens 10 such as "upper" and "lower" shall indicate the positional relationship for when the eyeglass lens 10 is processed for use in eyeglasses and worn. The vertical positional relationship of the eyeglass lens 10 shall coincide with the vertical positional relationship of the pages containing Figures 1 and 11.

As shown in Figure 1, the right-eye eyeglass lens 10R has a right-eye distance portion 11R, a right-eye near portion 12R formed in a different position from the right-eye distance portion 11R, and a right-eye progressive portion 13R formed between the right-eye distance portion 11R and the right-eye near portion 12R. For example, the right-eye distance portion 11R is formed in an upper part of the right-eye eyeglass lens 10R, the right-eye near portion 12R is formed in a lower part of the right-eye eyeglass lens 10R, and the right-eye progressive portion 13R is formed in an intermediate part of the right-eye eyeglass lens 10R. The right-eye distance portion 11R has refractive power suitable for distance viewing. The right-eye near portion 12R has refractive power suitable for near viewing. The right-eye progressive portion 13R is formed such that its refractive power continuously changes from the refractive power suitable for distance viewing in the vicinity of the right-eye distance portion 11R to the refractive power suitable for near viewing in the vicinity of the right-eye near portion 12R. In the embodiment, "diopter power" (the unit is the diopter (D)) is sometimes used as the numerical value representing refractive power.

As shown in Figure 1, the left-eye eyeglass lens 10L has a left-eye distance portion 11L, a left-eye near portion 12L formed in a different position from the left-eye distance portion 11L, and a left-eye progressive portion 13L formed between the left-eye distance portion 11L and the left-eye near portion 12L. For example, the left-eye distance portion 11L is formed in an upper part of the left-eye eyeglass lens 10L, the left-eye near portion 12L is formed in a lower part of the left-eye eyeglass lens 10L, and the left-eye progressive portion 13L is formed in an intermediate part of the left-eye eyeglass lens 10L. The left-eye distance portion 11L has refractive power suitable for distance viewing. The left-eye near portion 12L has refractive power suitable for near viewing. The left-eye progressive portion 13L is formed such that its refractive power continuously changes from the refractive power suitable for distance viewing in the vicinity of the left-eye distance portion 11L to the refractive power suitable for near viewing in the vicinity of the left-eye near portion 12L.

As can be seen from a sight line SR1 of the right eye ER and a sight line SL1 of the left eye EL in Figure 2, a wearer W of the pair of eyeglass lenses 1 uses a temporal portion of the right-eye eyeglass lens 10R (the right-eye distance portion 11R) and a nasal portion of the left-eye eyeglass lens 10L (the left-eye distance portion 11L) when viewing the right side of a distant object OB. As can be seen from a sight line SR2 of the right eye ER and a sight line SL2 of the left eye EL in Figure 2, the wearer W of the pair of eyeglass lenses 1 uses a nasal portion of the right-eye eyeglass lens 10R (the right-eye distance portion 11R) and a temporal portion of the left-eye eyeglass lens 10L (the left-eye distance portion 11L) when viewing the left side of the distant object OB.

The eyeglass lens 10, which is also referred to as the progressive refractive power lens, causes astigmatism since the refractive power changes depending on the part of the lens. To address this, the appearance of objects through the eyeglass lens 10 is improved by altering the distribution of astigmatism through design modifications. For example, the degree of blur when viewing the distant object OB is changed between the right eye ER and the left eye EL by altering the distribution of astigmatism between the temporal and nasal portions in the right-eye and left-eye distance portions 11R and 11L. However, the difference in the degree of blur that is tolerable when viewed by the wearer W with both eyes between the right eye ER and the left eye EL varies from person to person. In the embodiment, the eyeglass lens 10 is designed in accordance with the wearer W by evaluating the sensitivity of the wearer W to the difference in the degree of blur between the right eye ER and the left eye EL for manufacture. The degree of blur is sometimes referred to as the blur amount in the following description.

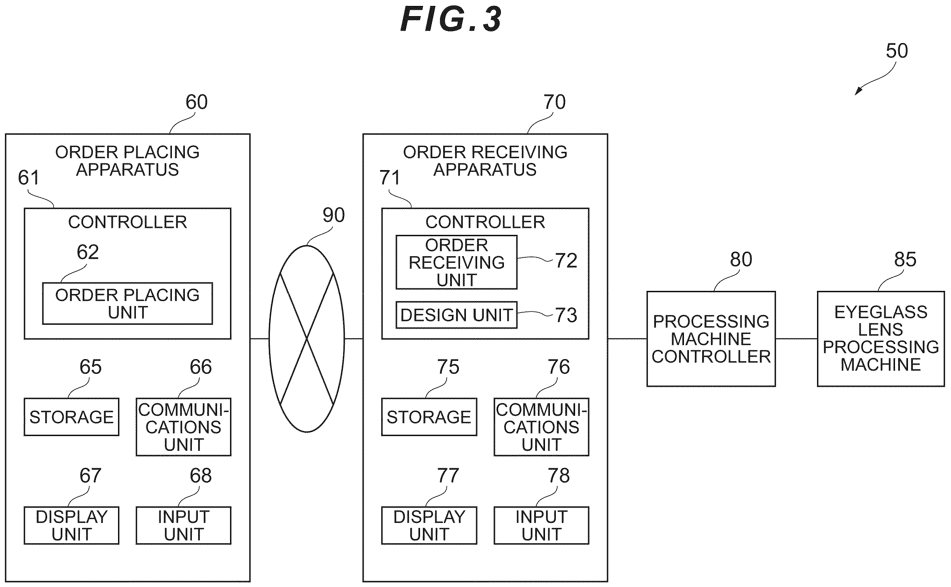

An eyeglass lens order placing and receiving system for manufacturing the pair of eyeglass lenses 1 of the embodiment will be described next. Figure 3 shows an eyeglass lens order placing and receiving system 50. As shown in Figure 3, this eyeglass lens order placing and receiving system 50 comprises an order placing apparatus 60 installed in an eyeglasses store (the order placing side), an order receiving apparatus 70 installed in a lens manufacturer, a processing machine controller 80, and an eyeglass lens processing machine 85. The order placing apparatus 60 and the order receiving apparatus 70 are communicably connected to each other via a network 90 such as the Internet. The order receiving apparatus 70 is connected with the processing machine controller 80, which is connected with the eyeglass lens processing machine 85. Note that only one order placing apparatus 60 is presented in Figure 3 for convenience of graphical illustration, but actually a plurality of order placing apparatuses 60 installed in a plurality of eyeglasses stores are connected to the order receiving apparatus 70.

The order placing apparatus 60 is a computer for placing an order for the eyeglass lens 10. The order placing apparatus 60 comprises a controller 61, a storage 65, a communications unit 66, a display unit 67, and an input unit 68. The controller 61 executes a program stored in the storage 65 and thereby controls the order placing apparatus 60. The controller 61 has an order placing unit 62 for placing an order for the eyeglass lens 10. The communications unit 66 communicates with the order receiving apparatus 70 via the network 90. The display unit 67 comprises a display device such as a CRT and an LCD display. The display unit 67 displays an order placement screen for inputting information on eyeglass lenses whose order is to be placed (order placement information), and the like. The input unit 68 comprises, for example, a mouse and a keyboard. For example, order placement information according to the content displayed on the order placement screen is input via the input unit 68. Note that the display unit 67 and the input unit 68 may be configured integrally by using a touch screen or the like.

The order receiving apparatus 70 is a computer for receiving an order for, designing, arithmetic processing of the optical performance of, and the like of the eyeglass lens 10. The order receiving apparatus 70 comprises a controller 71, a storage 75, a communications unit 76, a display unit 77, and an input unit 78. The controller 71 executes a program stored in the storage 75 and thereby controls the order receiving apparatus 70. The controller 71 has an order receiving unit 72 for receiving an order for the eyeglass lens 10, and a design unit 73 for designing the eyeglass lens 10. The storage 75 holds various data for designing eyeglass lenses in a readable manner. The communications unit 76 communicates with the order placing apparatus 60 via the network 90. The communications unit 76 also communicates with the processing machine controller 80. The display unit 77 comprises a display device such as a CRT and an LCD display. The display unit 77 displays a result of designing the eyeglass lens 10, and the like. The input unit 78 comprises, for example, a mouse and a keyboard. Note that the display unit 77 and the input unit 78 may be configured integrally by using a touch screen or the like.

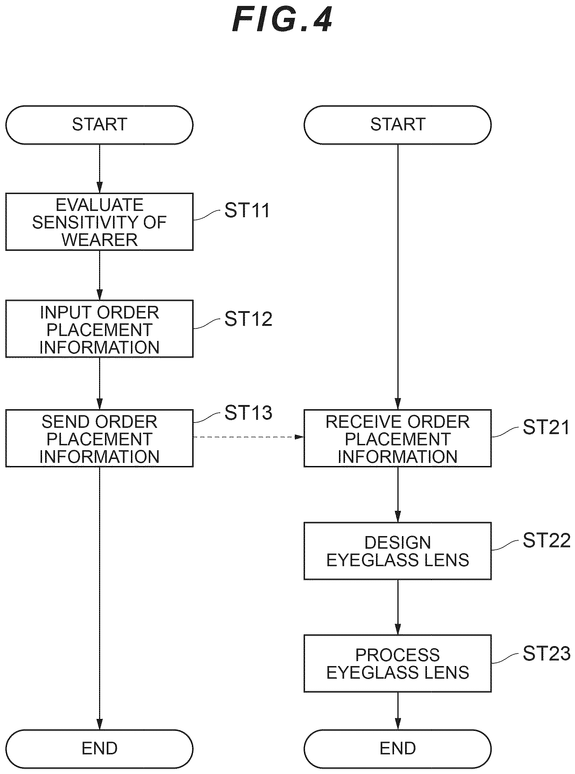

Procedures of manufacturing and providing the pair of eyeglass lenses 1 using the eyeglass lens order placing and receiving system 50 will be described next with reference to Figure 4. Figure 4 is a flowchart showing a flow of manufacture and provision of the pair of eyeglass lenses 1. Note that the left side of Figure 4 shows a procedure performed on the eyeglasses store side, and the right side of Figure 4 shows a procedure performed on the lens manufacturer side. An order placing party evaluates the sensitivity of a wearer (Step ST11). The sensitivity evaluation method will be described in detail later.

The order placing party next causes the display unit 67 of the order placing apparatus 60 to display the order placement screen, and inputs order placement information via the input unit 68 (Step ST12). The order placing party then determines order placement information of the eyeglass lenses to be ordered that includes information on sensitivity to the difference in blur amount between both eyes acquired when evaluating the sensitivity of the wearer.

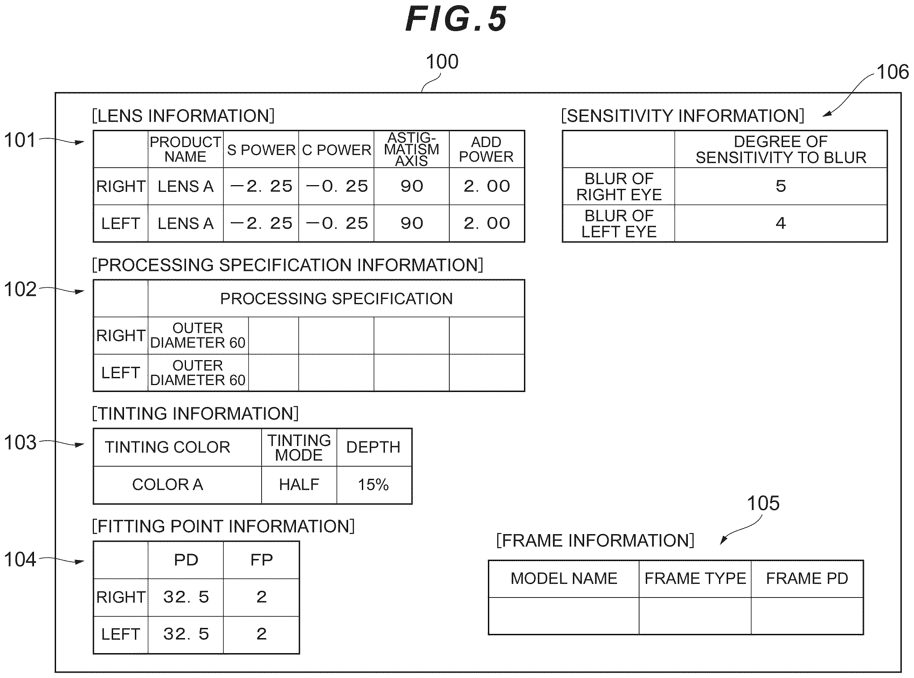

Figure 5 shows an example of the order placement screen 100. A Lens Information table 101 is input with Product name of the lenses to be ordered, and items related to the lens power to be ordered, including Spherical power (S power), Astigmatism power (C power), Astigmatism axis, and Add power. A Processing Specification Information table 102 is used for specifying the outer diameter and arbitrary point thickness of the lenses to be ordered. A Tinting Information table 103 is used for specifying the color of the lenses. Fitting Point (FP) Information 104 is input with the positional information of the eyes of the wearer W. PD represents the pupillary distance. A Frame Information table 105 is input with Frame model name, Frame type, and the like. A Sensitivity Information table 106 is input with numerical values representing the degree of sensitivity to blur as the information on sensitivity to the difference in blur amount between both eyes acquired when evaluating the sensitivity of the wearer. In the example shown in Figure 5, the degree of sensitivity to blur is expressed as ten levels of numerical values for each of the difference in the degree of blur of the right eye with respect to the left eye and the difference in the degree of blur of the left eye with respect to the right eye (the difference in the degree of blur of the right eye with respect to the left eye is expressed as "5," and the difference in the degree of blur of the left eye with respect to the right eye is expressed as "4"). The degree of sensitivity to blur is defined such that the larger the numeral, the stronger the sensitivity to blur.

Note that the sensitivity to blur may be expressed such that the smaller the sensitivity to blur, the larger the numeral, and may be expressed by symbols instead of numerical values. There is no limitation on how to express the sensitivity to blur as long as it can be expressed and informed according to a predetermined criterion.

After the order placing party inputs into each table on the order placement screen 100 shown in Figure 5 and clicks a send button (not shown), the order placing unit 62 of the order placing apparatus 60 acquires information input into each table on the order placement screen 100 (order placement information). The order placing apparatus 60 sends the order placement information acquired by the order placing unit 62 to the order receiving apparatus 70 via the communications unit 66 (Step ST13). Note that the processes, in the order placing apparatus 60, of displaying the order placement screen 100, acquiring the order placement information input through the order placement screen 100, and sending the order placement information to the order receiving apparatus 70 are performed by the controller 61 of the order placing apparatus 60 executing a specified program installed in advance in the storage 65.

After the order placement information is sent from the order placing apparatus 60, the order receiving unit 72 of the order receiving apparatus 70 receives the order placement information sent from the order placing apparatus 60 via the communications unit 76 (Step ST21). The design unit 73 of the order receiving apparatus 70 designs the eyeglass lens 10 based on the received order placement information (Step ST22). The method of designing the eyeglass lens 10 will be described in detail later. The order receiving apparatus 70 outputs design data of the eyeglass lens 10 designed by the design unit 73 to the processing machine controller 80 via the communications unit 76.

Based on the design data output from the order receiving apparatus 70, the processing machine controller 80 sends processing instructions to the eyeglass lens processing machine 85 (Step ST23). The eyeglass lens processing machine 85 thus processes and manufactures the eyeglass lenses based on the design data. The eyeglass lens 10 (i.e., the pair of eyeglass lenses 1) manufactured by the eyeglass lens processing machine 85 is shipped to the eyeglasses store, is fitted into the eyeglass frame, and is provided to the customer (the wearer W). Note that the processes, in the order receiving apparatus 70, of receiving the order placement information from the order placing apparatus 60, designing the eyeglass lenses based on the received order placement information, and outputting the design data of the eyeglass lenses to the processing machine controller 80 are performed by the controller 71 of the order receiving apparatus 70 executing a specified program installed in advance in the storage 75.

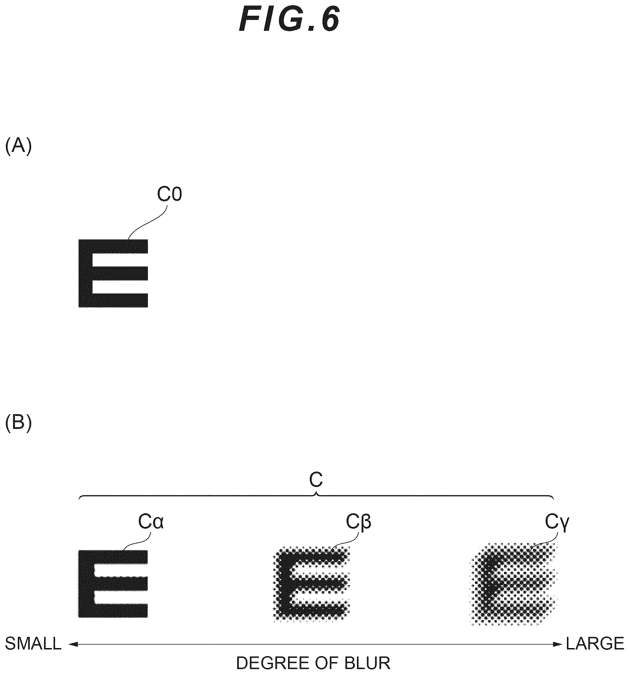

Next, the sensitivity evaluation method will be described. In the embodiment, a wearer's sensitivity to blur is evaluated by having the wearer's both eyes each view a plurality of blurred images C and/or an original image C0 (see Figure 6). Note that an image before being blurred is referred to as the original image C0. Figure 6 illustrates the original image C0 and the blurred images C. Figure 6 (A) shows the original image C0 consisting of the letter "E." Figure 6 (B) shows a plurality of blurred images Cα, Cβ, and Cγ generated from the original image C0 in Figure 6 (A) by blurring it to different degrees. The blurred image Cα has a slight distortion in the outline and the like, and its degree of blur is small. The blurred image Cβ has an outline that cannot be recognized clearly, and its degree of blur is moderate. The blurred image Cγ appears unclear overall, and its degree of blur is large.

The blurred images C are virtually-created perceived images of the original image C0 as viewed through an eyeglass lens with aberrations (astigmatism, refractive power errors, and the like). The degree of aberrations of the eyeglass lens corresponds to the degree of blur of the generated blurred images C. In the example shown in Figure 6, the degree of astigmatism of the eyeglass lens corresponds to the degree of blur of the blurred images C. Therefore, the astigmatism or other optical characteristics of the eyeglass lens 10 can be set appropriately for the wearer W on the basis of information on the wearer's sensitivity obtained with respect to the blurred images C having different degrees of blur.

An example of generating the blurred images C is to, in a model where the original image C0 is placed at a position a predetermined distance (the distance between the wearer and a blurred image in the sensitivity evaluation) away from the front surface of an eyeball and where the eyeglass lens is placed on the optical path from the original image C0 to the retina of the eyeball, perform ray tracing from one point in the original image C0 and determine the point spread function (PSF) on the retina. The blurred images C are then generated by convolving the luminance and color density of each point in the original image C0 using the point spread function. Note that the ray tracing computation can be performed by using, for example, a PC (Personal Computer). The blurred images C with different blur amounts can be generated by appropriately altering the aberrations (astigmatism, refractive power errors, and the like) of the eyeglass lens in the model for ray tracing.

For example, the blurred images C are generated with the minimum amount of aberration set to 0 D (i.e., the amount of aberration of the eyeglass lens corresponding to the original image C0), the maximum amount of aberration set to 1 D to 3 D, and the pitch interval of the amount of aberration set to 0.05 D to 0.25 D. This allows the corresponding amount of aberration of the eyeglass lens to be known in association with the blurred images C with different blur amounts.

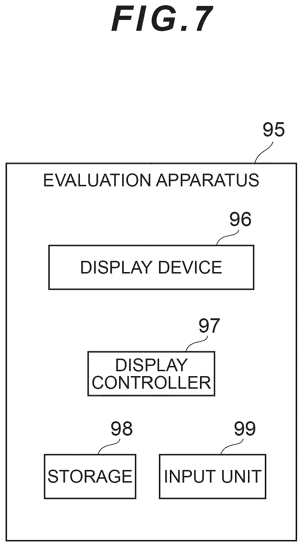

When the blurred images C or the original image C0 is made to be viewed, a display device 96 provided in an evaluation apparatus 95 (see Figure 7) is used to have both eyes of the wearer each view a different image. As schematically shown in Figure 7, the evaluation apparatus 95 comprises the display device 96, a display controller 97, a storage 98, and an input unit 99. The display device 96 is configured to be able to have both eyes of the wearer each view a different image at the same time using, for example, a head-mounted display or a polarized display using a polarizing filter (also called a three-dimensional display). The display controller 97 performs control to choose one of the blurred images C and the original image C0 and cause the display device 96 to display the one. The storage 98 holds image data of the blurred images C generated in advance and the original image C0 in a readable manner. The input unit 99 comprises, for example, a mouse and a keyboard. For example, information on the blurred images C or the original image C0 that the display device 96 is caused to display is input via the input unit 99.

The evaluation apparatus 95 may be incorporated into the order placing apparatus 60, or may be installed in the eyeglasses store (the order placing party) separately from the order placing apparatus 60. The evaluation apparatus 95 may also have an image generator (not shown) for generating the blurred images C. In the embodiment, the blurred images C are sometimes referred to as blurred images C1 to Cn (n is an integer of 2 or more). For example, when n = 5, blurred images C1 to C5 represent a blurred image C1 with the smallest blur amount, a blurred image C2 with the second smallest blur amount, a blurred image C3 with the third smallest blur amount, a blurred image C4 with the fourth smallest blur amount, and a blurred image C5 with the largest blur amount. Similarly, the blurred images C1 to Cn represent the blurred image C1 with the smallest blur amount, ..., and a blurred image Cn with the largest blur amount.

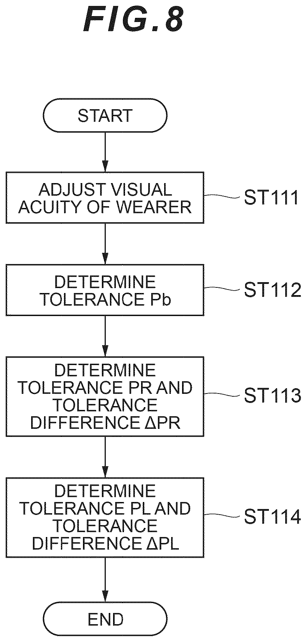

Figure 8 is a flowchart showing a procedure of evaluating the sensitivity of the wearer corresponding to Step ST11. When evaluating the sensitivity of the wearer, the order placing party uses a corrective lens or the like to adjust the visual acuity of the wearer so that the wearer can clearly view the original image C0 (Step ST111). In so doing, the order placing party may adjust the visual acuity of the wearer while having the wearer wear the display device 96. The configuration of the display device 96 using a head-mounted display, a polarized display (a three-dimensional display), or the like allows for adjusting the visual acuity of the wearer so that the wearer can clearly view the original image C0 at a position corresponding to the position the predetermined distance (the distance between the wearer and a blurred image in the sensitivity evaluation) away from the front surface of the eyeball via an eyepiece optical system or the like provided in the display device 96.

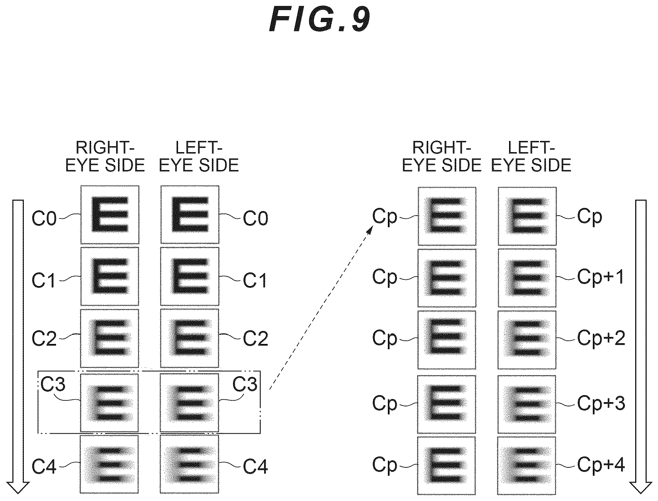

The order placing party then has the wearer wear the display device 96, has the wearer's both eyes each view the original image C0 or the blurred images C1 to Cn, and determines a tolerance Pb for astigmatism that is tolerable (i.e., does not cause discomfort to the wearer) when viewed by both eyes (Step ST112). Specifically, as shown in Figure 9, the wearer's both eyes are each made to view the original image C0, the blurred image C1 with the smallest blur amount, the blurred image C2 with the second smallest blur amount, the blurred image C3 with the third smallest blur amount, the blurred image C4 with the fourth smallest blur amount, ... in this order. During this process, the wearer viewing the blurred images is asked to answer whether the viewing with both eyes is tolerable or not verbally or by performing an operation to input into the input unit 99 of the evaluation apparatus 95. Then, the amount of aberration corresponding to the blurred image with the maximum blur amount (e.g., the blurred image C3 in Figure 9) among blurred images that are tolerable when viewed by both eyes (e.g., the blurred images C1 to C3 in Figure 9) is determined as the astigmatism tolerance Pb. This astigmatism tolerance Pb is sometimes referred to as the base tolerance Pb in the following description. Note that the order in which the original image C0 or the blurred images C1 to Cn are made to be viewed is not limited to the order from the original image C0 to increasing blur amounts, and may be an order from the blurred image Cn with the maximum blur amount to decreasing blur amounts, or a random order.

After determining the base tolerance Pb, the order placing party sets a blurred image corresponding to the aberration amount that is 1/3 to 2/3 of the base tolerance Pb among the blurred images C1 to Cn as the reference blurred image Cp. The order placing party has one of the wearer's both eyes (e.g., the right eye) view the reference blurred image Cp, has the other of the wearer's both eyes (e.g., the left eye) view a blurred image with the blur amount of the reference blurred image Cp or more, and determines a tolerance PR for astigmatism that is tolerable when viewed by both eyes (Step ST113). For example, as shown in Figure 9, the other eye (e.g., the left eye) is made to view the reference blurred image Cp, a blurred image Cp+1 with a blur amount that is larger than that of the reference blurred image Cp, a blurred image Cp+2 with a blur amount that is larger than that of the blurred image Cp+1, a blurred image Cp+3 with a blur amount that is larger than that of the blurred image Cp+2, a blurred image Cp+4 with a blur amount that is larger than that of the blurred image Cp+3, ... in this order. During this process, the wearer viewing the blurred images is asked to answer whether the viewing with both eyes is tolerable or not verbally or by performing an operation to input into the input unit 99 of the evaluation apparatus 95. Then, the amount of aberration corresponding to the blurred image with the maximum blur amount that is tolerable when viewed by both eyes among the blurred images Cp+1, Cp+2, ... made to be viewed by the other eye (e.g., the left eye) is determined as the astigmatism tolerance PR. The difference between the determined astigmatism tolerance PR and the base tolerance Pb, ΔPR, is also determined. This difference ΔPR between the astigmatism tolerance PR and the base tolerance Pb is sometimes referred to as the first tolerance difference ΔPR in the following description.

The order placing party then has the other of the wearer's both eyes (e.g., the left eye) view the reference blurred image Cp, has the one of the wearer's both eyes (e.g., the right eye) view a blurred image with the blur amount of the reference blurred image Cp or more, and determines a tolerance PL for astigmatism that is tolerable when viewed by both eyes (Step ST114). At this time, as in previous Step ST113, the amount of aberration corresponding to the blurred image with the maximum blur amount that is tolerable when viewed by both eyes among the blurred images Cp+1, Cp+2, ... made to be viewed by the one eye (e.g., the right eye) is determined as the astigmatism tolerance PL. The difference between the determined astigmatism tolerance PL and the base tolerance Pb, ΔPL, is also determined. This difference ΔPL between the astigmatism tolerance PL and the base tolerance Pb is sometimes referred to as the second tolerance difference ΔPL in the following description.

Note that the reference blurred image Cp or the blurred images Cp+1, Cp+2, ... may be used by choosing from among the blurred images C1 to Cn used in previous Step ST112, or may be newly generated according to the base tolerance Pb. If those are used by choosing from among the blurred images C1 to Cn used in previous Step ST112, the blurred image C3 with the third smallest blur amount is set as the reference blurred image Cp in the example shown in Figure 9. The order in which the reference blurred image Cp or the blurred images Cp+1, Cp+2, ... are made to be viewed is not limited to the order from the reference blurred image Cp to increasing blur amounts, and may be an order from the blurred image with the maximum blur amount to decreasing blur amounts, or a random order.

In this way, information on the first tolerance difference ΔPR and the second tolerance difference ΔPL is acquired as information on sensitivity to the difference in blur amount between both eyes. After the processes to evaluate the sensitivity of the wearer (Steps ST111 to ST114) are completed, the procedure proceeds to the process of inputting order placement information (Step ST12). Note that, in the example of the order placement screen 100 shown in Figure 5, the numerical values representing the degree of sensitivity to blur are expressed by ten levels of numerical values based on the first tolerance difference ΔPR and the second tolerance difference ΔPL.

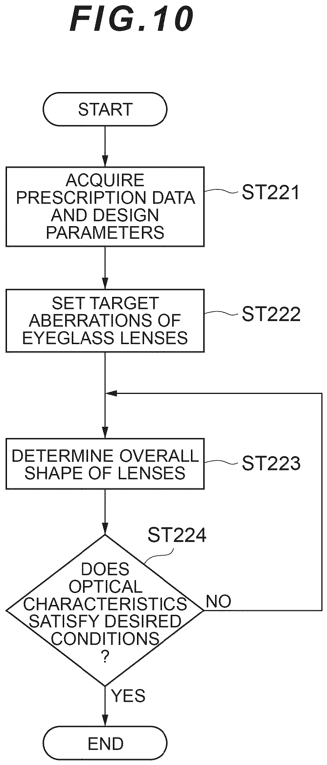

Next, the method of designing the eyeglass lens 10 will be described. Figure 10 is a flowchart showing a procedure of designing the eyeglass lens 10 corresponding to Step ST22. When designing the eyeglass lens 10, the order receiving apparatus 70 acquires prescription data for the eyeglass lenses, and design parameters including information on sensitivity to the difference in blur amount between both eyes (e.g., the information on the first tolerance difference ΔPR and the second tolerance difference ΔPL described above)(Step ST221). When doing so, the order receiving apparatus 70 also acquires the pantoscopic tilt of the frame, the wrap angle, the distance between the eyes and the lenses, and other fitting parameters and the like as appropriate.

The design unit 73 of the order receiving apparatus 70 then sets target aberrations of the eyeglass lenses based on the design parameters including information on sensitivity to the difference in blur amount between both eyes acquired in previous Step ST221 (Step ST222). As described earlier, the degree of blur when viewing a distant object can be changed between the right and left eyes by altering the distribution of astigmatism between the temporal and nasal portions, that is, by altering the symmetry of the distribution of astigmatism, in the distance portions of the eyeglass lenses which are also referred to as progressive refractive power lenses. Here, let De be a design parameter for adjusting the aberration amount of temporal astigmatism in a distance portion of an eyeglass lens. Let Dn be a design parameter for adjusting the aberration amount of nasal astigmatism in a distance portion of an eyeglass lens. The design parameters De and Dn are defined such that the greater the value, the wider the area with small astigmatism, that is, the area with clear vision.

A design condition in which De = +5 and Dn = -5 is referred to as a first lens design condition. A design condition in which De = 0 and Dn = 0 is referred to as a second lens design condition. A design condition in which De = -5 and Dn = +5 is referred to as a third lens design condition. Figure 11 (A) shows the distribution of astigmatism in the eyeglass lens 10 designed with the first lens design condition. Figure 11 (B) shows the distribution of astigmatism in the eyeglass lens 10 designed with the second lens design condition. Figure 11 (C) shows the distribution of astigmatism in the eyeglass lens 10 designed with the third lens design condition. In Figures 11 (A) to (C), the areas that appear darker indicate areas with larger astigmatism. As can be seen from Figures 11 (A) to (C), the symmetry of the distribution of astigmatism in the eyeglass lens 10 can be altered by changing the design parameters De and Dn.

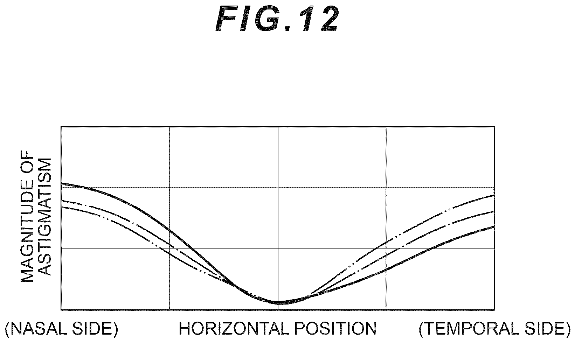

Figure 12 shows the magnitude of astigmatism in a distance portion of an eyeglass lens for each lens design condition for comparison. The solid line in the graph shown in Figure 12 represents the magnitude of astigmatism when the lens is designed with the first lens design condition. The dash-dot line in the graph shown in Figure 12 represents the magnitude of astigmatism when the lens is designed with the second lens design condition. The dash-dot-dot line in the graph shown in Figure 12 represents the magnitude of astigmatism when the lens is designed with the third lens design condition. With the first lens design condition, as can be seen from Figure 12, the temporal astigmatism in the distance portion of the eyeglass lens becomes relatively small and the nasal astigmatism becomes relatively large. With the third lens design condition, on the other hand, the temporal astigmatism in the distance portion of the eyeglass lens becomes relatively large and the nasal astigmatism becomes relatively small.

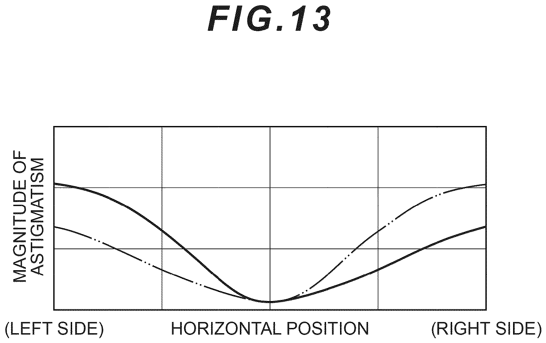

Figure 13 shows the magnitude of astigmatism in the distance portions of the eyeglass lenses designed with the first lens design condition. The solid line in the graph shown in Figure 13 represents the magnitude of astigmatism in the right-eye distance portion 11R of the right-eye eyeglass lens 10R (see Figure 1) when the lens is designed with the first lens design condition. The dash-dot-dot line in the graph shown in Figure 13 represents the magnitude of astigmatism in the left-eye distance portion 11L of the left-eye eyeglass lens 10L (see Figure 1) when the lens is designed with the first lens design condition. With the first lens design condition, as can be seen from Figure 13, the difference in astigmatism between the right-eye eyeglass lens 10R (the right-eye distance portion 11R) and the left-eye eyeglass lens 10L (the left-eye distance portion 11L) becomes large.

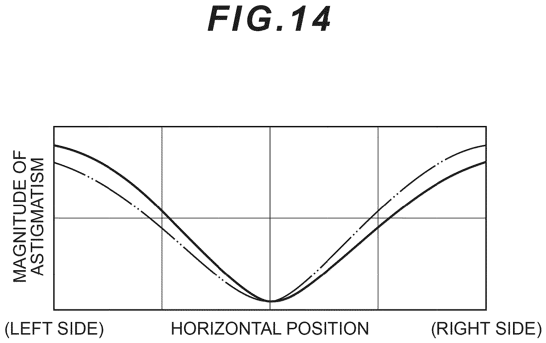

Figure 14 shows the magnitude of astigmatism in the distance portions of the eyeglass lenses designed with the second lens design condition. The solid line in the graph shown in Figure 14 represents the magnitude of astigmatism in the right-eye distance portion 11R of the right-eye eyeglass lens 10R (see Figure 1) when the lens is designed with the second lens design condition. The dash-dot-dot line in the graph shown in Figure 14 represents the magnitude of astigmatism in the left-eye distance portion 11L of the left-eye eyeglass lens 10L (see Figure 1) when the lens is designed with the second lens design condition. With the second lens design condition, as can be seen from Figure 14, the difference in astigmatism between the right-eye eyeglass lens 10R (the right-eye distance portion 11R) and the left-eye eyeglass lens 10L (the left-eye distance portion 11L) becomes small.

In the embodiment, target aberrations of the eyeglass lenses are set based on the first tolerance difference ΔPR and the second tolerance difference ΔPL described earlier. Specifically, when the first tolerance difference ΔPR and the second tolerance difference ΔPL are large, the target aberrations are set such that the difference in astigmatism between the right-eye eyeglass lens 10R (the right-eye distance portion 11R) and the left-eye eyeglass lens 10L (the left-eye distance portion 11L) becomes large, as with the first lens design condition. On the other hand, when the first tolerance difference ΔPR and the second tolerance difference ΔPL are small, the target aberrations are set such that the difference in astigmatism between the right-eye eyeglass lens 10R (the right-eye distance portion 11R) and the left-eye eyeglass lens 10L (the left-eye distance portion 11L) becomes small, as with the second lens design condition. In other words, the target aberrations are set such that, the larger the first tolerance difference ΔPR and the second tolerance difference ΔPL become, the smaller the temporal astigmatism in the right-eye distance portion 11R of the right-eye eyeglass lens 10R becomes compared to the nasal astigmatism in the left-eye distance portion 11L of the left-eye eyeglass lens 10L, and the smaller the temporal astigmatism in the left-eye distance portion 11L of the left-eye eyeglass lens 10L becomes compared to the nasal astigmatism in the right-eye distance portion 11R of the right-eye eyeglass lens 10R.

When the target aberrations are set such that the difference in astigmatism becomes large as with the first lens design condition, the area with small aberrations becomes wide on the right side, that is, the temporal portion, of the right-eye distance portion 11R of the right-eye eyeglass lens 10R as shown in Figure 13. Accordingly, the area through which a distant object can be recognized only by the right eye like monovision becomes wide. The area with small aberrations becomes wide also on the left side, that is, the temporal portion, of the left-eye distance portion 11L of the left-eye eyeglass lens 10L. Accordingly, the area through which a distant object can be recognized only by the left eye like monovision becomes wide. However, since the aberration amount of astigmatism is different between the temporal and nasal portions in the distance portions of the eyeglass lenses, the blur amount when viewing the right side or left side of a distant object differs between both eyes. Therefore, this setting results in an eyeglass lens design that causes discomfort to wearers whose first tolerance difference ΔPR and second tolerance difference ΔPL are small, that is, who are likely to have discomfort when there is a difference in appearance (blur amount) between both eyes.

On the other hand, when the target aberrations are set such that the difference in astigmatism becomes small as with the second lens design condition, the distribution of astigmatism in the distance portions of the eyeglass lenses becomes nearly bilaterally symmetrical, as shown in Figure 14. This allows appearance (blur amount) when viewing the right side or left side of a distant object to be almost the same for both eyes, making it less likely to cause discomfort. However, this setting causes a feeling that the area of clear vision is smaller than the first lens design condition in wearers whose first tolerance difference ΔPR and second tolerance difference ΔPL are large, that is, who are not likely to have discomfort even if there is a difference in appearance (blur amount) between both eyes.

The design parameters De and Dn can be calculated based on the first tolerance difference ΔPR and the second tolerance difference ΔPL. First, a case will be described in which the same set of design parameters De and Dn is used for the right-eye eyeglass lens 10R and the left-eye eyeglass lens 10L to simplify the calculation. In this case, the design parameter De is expressed by the following expression (1), and the design parameter Dn is expressed by the following expression (2). $De = D 0 + ΔP ⋅ k$ $Dn = D 0 - ΔP ⋅ k$

Here, ΔP is the arithmetic average of the first tolerance difference ΔPR and the second tolerance difference ΔPL. D0 is a design parameter that is set when the same design parameter is used for the temporal and nasal sides in, for example, adjusting astigmatism. k is a predetermined coefficient. D0 and k vary depending on the prescription data for the eyeglass lenses and the limit of tolerable astigmatism (blur). Note that wearers use temporal portions of eyeglass lenses more than those nasal portions, and therefore the design parameter De for the temporal portions of the eyeglass lenses is set to be larger than the design parameter Dn for those nasal portions.

Next, a case will be described in which different design parameters are used for the right-eye eyeglass lens 10R and the left-eye eyeglass lens 10L. In this case, a design parameter DRe for adjusting the aberration amount of temporal astigmatism in the right-eye distance portion 11R of the right-eye eyeglass lens 10R is expressed by the following expression (3), and a design parameter DRn for adjusting the aberration amount of nasal astigmatism in it is expressed by the following expression (4). $DRe = D 0 + ΔPR ⋅ k$ $DRn = D 0 - ΔPL ⋅ k$

A design parameter DLe for adjusting the aberration amount of temporal astigmatism in the left-eye distance portion 11L of the left-eye eyeglass lens 10L is expressed by the following expression (5), and a design parameter DLn for adjusting the aberration amount of nasal astigmatism in it is expressed by the following expression (6). $DLe = D 0 + ΔPL ⋅ k$ $DLn = D 0 - ΔPR ⋅ k$

Here, D0 and k are the same as those described in relation to the above expressions (1) and (2).

After setting the target aberrations of the eyeglass lenses, the order receiving apparatus 70 determines the overall shape of the eyeglass lenses (Step ST223). After determining the overall shape of the eyeglass lenses, the order receiving apparatus 70 determines whether the refractive power, astigmatism, or other optical characteristics of the eyeglass lenses satisfy desired conditions or not (Step ST224). If the optical characteristics do not satisfy the desired conditions, that is, if the determination at Step ST224 is NO, the process returns to previous Step ST223. If the optical characteristics satisfy the desired conditions, that is, if the determination at Step ST224 is YES, the process of designing the eyeglass lenses ends. After the end of the steps of designing the eyeglass lenses (Steps ST221 to ST224), the procedure proceeds to the process of processing the eyeglass lenses (Step ST23).

In the embodiment, a wearer's right and left eyes are each made to view an image with different blur amounts (e.g., the original image C0 or the blurred images C1 to Cn), and information on sensitivity to the difference in blur amount between the images that the right and left eyes were each made to view is acquired. This allows for evaluating the wearer's sensitivity to the difference in appearance (blur amount) between both eyes, and appropriately designing the eyeglass lenses based on the acquired information on the wearer's sensitivity. For example, one of a wearer's both eyes is made to view the reference blurred image (the first image) Cp with a predetermined blur amount (the blur amount corresponding to the reference blurred image Cp), and the other of the wearer's both eyes is made to view blurred images (the second image) Cp+1, Cp+2, ... with blur amounts larger than that of the reference blurred image Cp. Then, information on sensitivity to the difference in blur amount between the blurred images Cp+1, Cp+2, ... made to be viewed by the other eye and the reference blurred image Cp made to be viewed by the one eye is acquired. Specifically, the amount of aberration corresponding to the blurred image with the maximum blur amount that is tolerable when viewed by both eyes among the blurred images Cp+1, Cp+2, ... made to be viewed by the other eye is determined as the astigmatism tolerance PR, and the difference between the astigmatism tolerance PR and the base tolerance Pb (i.e., the amount of aberration corresponding to the reference blurred image Cp), ΔPR (the first tolerance difference ΔPR), is determined.

Additionally, the other eye is made to view the reference blurred image Cp, and the one eye is made to view the blurred images Cp+1, Cp+2, ... Then, information on sensitivity to the difference in blur amount between the blurred images Cp+1, Cp+2, ... made to be viewed by the one eye and the reference blurred image Cp made to be viewed by the other eye is acquired. Specifically, the amount of aberration corresponding to the blurred image with the maximum blur amount that is tolerable when viewed by both eyes among the blurred images Cp+1, Cp+2, ... made to be viewed by the one eye is determined as the astigmatism tolerance PL, and the difference between the astigmatism tolerance PL and the base tolerance Pb, ΔPL (the second tolerance difference ΔPL), is determined. This allows for accurately evaluating the wearer's sensitivity to the difference in appearance (blur amount) between both eyes, and appropriately designing the eyeglass lenses based on the acquired information on the wearer's sensitivity (i.e., the information on the first tolerance difference ΔPR and the second tolerance difference ΔPL).

In the embodiment, both one and the other eyes are each made to view the blurred images (the third image) C1 to Cn with the same blur amounts, and information on sensitivity to the blur amount of the blurred images C1 to Cn is acquired. Specifically, the amount of aberration corresponding to the blurred image with the maximum blur amount among blurred images that are tolerable when viewed by both eyes is determined as the astigmatism tolerance Pb (the base tolerance Pb). Then, the reference blurred image Cp (i.e., the predetermined blur amount corresponding to the reference blurred image Cp) is set based on the base tolerance Pb. This allows for accurately evaluating the wearer's sensitivity to the difference in appearance (blur amount) between both eyes, and appropriately designing the eyeglass lenses.

In the embodiment, the right-eye eyeglass lens 10R and the left-eye eyeglass lens 10L are designed such that, the larger the maximum value of the difference in blur amount that is tolerable when viewed by both eyes, that is, the first tolerance difference ΔPR and the second tolerance difference ΔPL, becomes, the smaller the temporal aberration in the right-eye distance portion 11R of the right-eye eyeglass lens 10R becomes compared to the nasal aberration in the left-eye distance portion 11L of the left-eye eyeglass lens 10L, and the smaller the temporal aberration in the left-eye distance portion 11L of the left-eye eyeglass lens 10L becomes compared to the nasal aberration in the right-eye distance portion 11R of the right-eye eyeglass lens 10R. This allows for appropriately designing and manufacturing the eyeglass lens 10 in accordance with the wearer's sensitivity to the difference in appearance (blur amount) between both eyes.

As described earlier, the right-eye eyeglass lens 10R has: a right-eye near portion 12R with refractive power suitable for near viewing; and a right-eye progressive portion 13R that is provided between the right-eye distance portion 11R and the right-eye near portion 12R and whose refractive power changes between the refractive power of the right-eye distance portion 11R and the refractive power of the right-eye near portion 12R. The left-eye eyeglass lens 10L has: a left-eye near portion 12L with refractive power suitable for near viewing; and a left-eye progressive portion 13L that is provided between the left-eye distance portion 11L and the left-eye near portion 12L and whose refractive power changes between the refractive power of the left-eye distance portion 11L and the refractive power of the left-eye near portion 12L. As seen above, the eyeglass lens 10 is preferably a progressive refractive power lens.

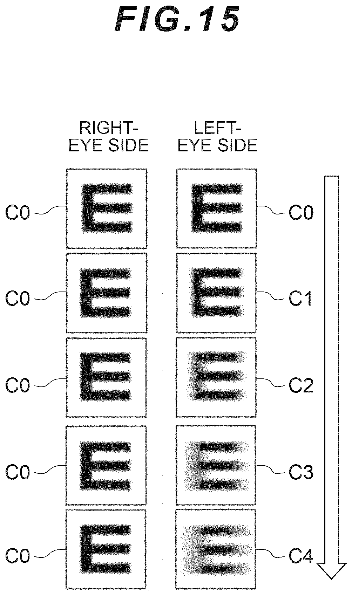

While in the embodiment described above the wearer's both eyes are each made to view the original image C0 or the blurred images C1 to Cn, and the tolerance Pb (the base tolerance Pb) for astigmatism that is tolerable when viewed by both eyes is determined in Step ST112, the disclosure is not limited to this. For example, as shown in Figure 15, Step ST112 may be omitted by setting the original image C0 as the reference blurred image Cp. This allows for evaluating the wearer's sensitivity to the difference in appearance (blur amount) between both eyes in a simple and easy manner.

In this case, the order placing party has one of the wearer's both eyes (e.g., the right eye) view the original image C0, has the other of the wearer's both eyes (e.g., the left eye) view the original image C0 or the blurred images C1 to Cn, and determines the tolerance PR for astigmatism that is tolerable when viewed by both eyes in Step ST113. For example, as shown in Figure 15, the other eye (e.g., the left eye) is made to view the original image C0, the blurred image C1 with the smallest blur amount, the blurred image C2 with the second smallest blur amount, the blurred image C3 with the third smallest blur amount, the blurred image C4 with the fourth smallest blur amount, ... in this order. During this process, the wearer viewing the blurred images is asked to answer whether the viewing with both eyes is tolerable or not verbally or by performing an operation to input into the input unit 99 of the evaluation apparatus 95. Then, the amount of aberration corresponding to the blurred image with the maximum blur amount that is tolerable when viewed by both eyes among the blurred images C1 to Cn made to be viewed by the other eye (e.g., the left eye) is determined as an astigmatism tolerance PR0. The difference between the determined astigmatism tolerance PR0 and the aberration amount corresponding to the original image C0, ΔPR (the first tolerance difference ΔPR), is also determined. Since the aberration amount corresponding to the original image C0 is zero, the first tolerance difference ΔPR becomes equal to the astigmatism tolerance PR0.

Also in this case, the order placing party has the other of the wearer's both eyes (e.g., the left eye) view the original image C0, has the one of the wearer's both eyes (e.g., the right eye) view the original image C0 or the blurred images C1 to Cn, and determines the tolerance PL0 for astigmatism that is tolerable when viewed by both eyes in Step ST114. During this process, as in previous Step ST113, the amount of aberration corresponding to the blurred image with the maximum blur amount that is tolerable when viewed by both eyes among the blurred images C1 to Cn made to be viewed by the one eye (e.g., the right eye) is determined as the astigmatism tolerance PL0. The difference between the determined astigmatism tolerance PL0 and the aberration amount corresponding to the original image C0, ΔPL (the second tolerance difference ΔPL), is also determined. Since the aberration amount corresponding to the original image C0 is zero, the second tolerance difference ΔPL also becomes equal to the astigmatism tolerance PL0. Note that the order in which the original image C0 or the blurred images C1 to Cn are made to be viewed is not limited to the order from the original image C0 to increasing blur amounts, and may be an order from the blurred image Cn with the maximum blur amount to decreasing blur amounts, or a random order.

While in the embodiment described above a wearer's both eyes are made to view the original image C0 or the blurred images C1 to Cn, or the reference blurred image Cp or the blurred images Cp+1, Cp+2, ..., the disclosure is not limited to this. For example, a wearer's both eyes may be made to view images with blur introduced to a predetermined image (e.g., the original image C0) by imparting aberrations to actual lenses such as a phoropter (e.g., images comparable to the blurred images C1 to Cn, or to the reference blurred image Cp or the blurred images Cp+1, Cp+2, ...).

While in the embodiment described above the one of both eyes is illustrated as the right eye and the other of both eyes is illustrated as the left eye, the disclosure is not limited to this, and the one of both eyes may be the left eye and the other of both eyes may be the right eye.

### DESCRIPTION OF THE SYMBOLS

1: One pair of eyeglass lenses
10R: Right-eye eyeglass lens
10L: Left-eye eyeglass lens

## Claims

1. A sensitivity evaluation method comprising:
having a right eye and a left eye each view an image with different blur amounts; and
acquiring information on sensitivity to a difference in blur amount between the images that the right and left eyes were each made to view.

2. The sensitivity evaluation method according to claim 1, comprising:
having one of the right and left eyes view a first image with a predetermined blur amount and having the other of the right and left eyes view a second image with a blur amount that is larger than the predetermined blur amount;
acquiring information on sensitivity to a difference in blur amount between the second image that the other eye was made to view and the first image that the one eye was made to view;
having the other eye view the first image with the predetermined blur amount and having the one eye view the second image with a blur amount that is larger than the predetermined blur amount; and
acquiring information on sensitivity to a difference in blur amount between the second image that the one eye was made to view and the first image that the other eye was made to view.

3. The sensitivity evaluation method according to claim 2, comprising:
having the right and left eyes each view a third image with a same blur amount and acquiring information on sensitivity to the blur amount of the third image that the right and left eyes were each made to view; and
setting the predetermined blur amount based on the information on sensitivity to the blur amount of the third image.

4. The sensitivity evaluation method according to claim 3, wherein the information on sensitivity to the blur amount of the third image is on a maximum value of the blur amount of the third image that is tolerable when viewed by both the right and left eyes.

5. The sensitivity evaluation method according to claim 2, comprising setting the predetermined blur amount to zero.

6. The sensitivity evaluation method according to any one of claims 1 to 5, wherein the information on sensitivity to the difference in blur amount is on a maximum value of the difference in blur amount that is tolerable when viewed by both the right and left eyes.

7. A method for manufacturing a pair of eyeglass lenses consisting of a right-eye eyeglass lens for use on a right eye and a left-eye eyeglass lens for use on a left eye, the method comprising:
having a right eye and a left eye each view an image with different blur amounts;
acquiring information on sensitivity to a difference in blur amount between the images that the right and left eyes were each made to view;
designing the right-eye and left-eye eyeglass lenses based on the information on sensitivity to the difference in blur amount; and
manufacturing the right-eye and left-eye eyeglass lenses based on the design.

8. The method for manufacturing a pair of eyeglass lenses according to claim 7, comprising:
having one of the right and left eyes view a first image with a predetermined blur amount and having the other of the right and left eyes view a second image with a blur amount that is larger than the predetermined blur amount;
acquiring information on sensitivity to a difference in blur amount between the second image that the other eye was made to view and the first image that the one eye was made to view;
having the other eye view the first image with the predetermined blur amount and having the one eye view the second image with a blur amount that is larger than the predetermined blur amount; and
acquiring information on sensitivity to a difference in blur amount between the second image that the one eye was made to view and the first image that the other eye was made to view,
wherein the right-eye and left-eye eyeglass lenses are designed based on the information on sensitivity to the difference in blur amount between the second image that the other eye was made to view and the first image that the one eye was made to view and the information on sensitivity to the difference in blur amount between the second image that the one eye was made to view and the first image that the other eye was made to view.

9. The method for manufacturing a pair of eyeglass lenses according to claim 7 or 8,
wherein the information on sensitivity to the difference in blur amount is on a maximum value of the difference in blur amount that is tolerable when viewed by both the right and left eyes, and
wherein the right-eye and left-eye eyeglass lenses are designed such that, the larger the maximum value of the difference in blur amount that is tolerable when viewed by both eyes becomes, the smaller a temporal aberration of the right-eye eyeglass lens becomes compared to a nasal aberration of the left-eye eyeglass lens, and the smaller a temporal aberration of the left-eye eyeglass lens becomes compared to a nasal aberration of the right-eye eyeglass lens.

10. The method for manufacturing a pair of eyeglass lenses according to claim 9,
wherein the right-eye eyeglass lens has a right-eye distance portion with refractive power suitable for distance viewing,
wherein the left-eye eyeglass lens has a left-eye distance portion with refractive power suitable for distance viewing, and
wherein the right-eye and left-eye eyeglass lenses are designed such that, the larger the maximum value of the difference in blur amount that is tolerable when viewed by both eyes becomes, the smaller a temporal aberration of the right-eye distance portion becomes compared to a nasal aberration of the left-eye distance portion, and the smaller a temporal aberration of the left-eye distance portion becomes compared to a nasal aberration of the right-eye distance portion.

11. The method for manufacturing a pair of eyeglass lenses according to claim 10,
wherein the right-eye eyeglass lens has: a right-eye near portion with refractive power suitable for near viewing; and a right-eye progressive portion that is provided between the right-eye distance portion and the right-eye near portion and whose refractive power changes between the refractive power of the right-eye distance portion and the refractive power of the right-eye near portion, and
wherein the left-eye eyeglass lens has: a left-eye near portion with refractive power suitable for near viewing; and a left-eye progressive portion that is provided between the left-eye distance portion and the left-eye near portion and whose refractive power changes between the refractive power of the left-eye distance portion and the refractive power of the left-eye near portion.
